# EUROPEAN PATENT APPLICATION

(11) **EP 2 602 315 A1**
(43) Date of publication of application: **12.06.2013**
(21) Application number: 12195089.3
(22) Date of filing: 30.11.2012
(51) Int. Cl.: C12N 5/00

(54) **Leukocyte purification**

(30) Priority: 05.12.2011 US 201113311046
(71) Applicant: Pall Corporation, Port Washington, NY 11050 (US)
(72) Inventor: Fomovsky, Mikhail, Port Washington, NY 11050 (US); Bormann, Thomas J., Huntington, NY 11743 (US)
(74) Representative: Hoeger, Stellrecht & Partner Patentanwälte

(57) **Abstract**

A method for purifying leukocytes is disclosed. The method can further include recovering the purified leukocytes, or lysing the purified leukocytes.

## Description

### BACKGROUND OF THE INVENTION

Leukocytes (also referred to as white blood cells) are useful in a variety of applications, including cell therapy and diagnostics. Leukocytes can be obtained from, for example, cord blood or blood obtained from a donor.

Conventionally, cord blood concentrates and stem cell concentrates, which include leukocytes, are stored under liquid nitrogen conditions, wherein the concentrates include the cryoprotectant dimethyl sulfoxide (DMSO) to protect the leukocytes from damage during storage. The concentrates including DMSO can be administered to a patient, but since DMSO can be considered an impurity, some protocols include centrifuging and washing the concentrates to reduce the presence of DMSO in the concentrate product before administration to a patient. Such protocols are labor intensive and time consuming.

Some methods for obtaining leukocytes include passing blood or blood products through a porous leukocyte depletion medium and either eluting the leukocytes from the medium by passing an elution fluid from the downstream surface of the medium through the upstream surface; or lysing the leukocytes on the medium after forcing saline through the medium from the upstream surface through the downstream surface or forcing air through the medium from the upstream surface through the downstream surface. However, these methods have resulted in an undesirable level of impurities, such as the presence of erythrocytes (also known as red blood cells) and/or a lower than desired yield of leukocytes.

The present invention provides for ameliorating at least some of the disadvantages of the prior art. These and other advantages of the present invention will be apparent from the description as set forth below.

### BRIEF SUMMARY OF THE INVENTION

An embodiment of the invention provides a method for purifying leukocytes, the method comprising (a) passing a biological fluid comprising leukocytes through a porous leukocyte depletion medium having an upstream surface and a downstream surface, the fluid passing from the upstream surface through the downstream surface, wherein leukocytes are retained by the leukocyte depletion medium; (b) passing a wash fluid through the leukocyte depletion medium from the downstream surface through the upstream surface; and, (c) passing the wash fluid through the leukocyte depletion medium from the upstream surface through the downstream surface via gravity.

Another embodiment provides a method for purifying leukocytes, the method comprising (a) passing a cryoprotectant fluid comprising leukocytes through a porous leukocyte depletion medium having an upstream surface and a downstream surface, the fluid passing from the upstream surface through the downstream surface, wherein leukocytes are retained by the leukocyte depletion medium; (b) passing a wash fluid through the leukocyte depletion medium from the downstream surface through the upstream surface; and, (c) passing the wash fluid through the leukocyte depletion medium from the upstream surface through the downstream surface via gravity.

In some embodiments of the methods, (b) and (c) are repeated at least once with additional wash fluid.

Some embodiments of the methods further comprise eluting leukocytes from the leukocyte depletion medium by passing an elution fluid through the leukocyte depletion medium from the downstream surface through the upstream surface, and recovering the eluted leukocytes, or lysing the leukocytes while retained by the leukocyte depletion medium.

If desired, embodiments of the method can be carried out using multiwell device, or individual filter devices.

### BRIEF DESCRIPTION OF THE SEVERAL VIEWS OF THE DRAWING(S)

Figure 1 describes, and illustrates diagrammatically, an embodiment of a method according to the present invention.

Figure 2 shows an illustrative system suitable for carrying out an embodiment of a method according to the invention.

Figure 3 shows another illustrative system suitable for carrying out an embodiment of a method according to the invention, wherein the method is carried out while maintaining a closed system.

### DETAILED DESCRIPTION OF THE INVENTION

In accordance with an embodiment of the present invention, a method for purifying leukocytes is provided, the method comprising (a) passing a biological fluid comprising leukocytes through a porous leukocyte depletion medium having an upstream surface and a downstream surface, the fluid passing from the upstream surface through the downstream surface, wherein leukocytes are retained by the leukocyte depletion medium; (b) passing a wash fluid through the leukocyte depletion medium from the downstream surface through the upstream surface; and, (c) passing the wash fluid through the leukocyte depletion medium from the upstream surface through the downstream surface via gravity.

Another embodiment of a method for purifying leukocytes comprises (a) passing a fluid comprising leukocytes and a cryoprotectant (such as DMSO) through a porous leukocyte depletion medium having an upstream surface and a downstream surface, the fluid passing from the upstream surface through the downstream surface, wherein leukocytes are retained by the leukocyte depletion medium; (b) passing a wash fluid through the leukocyte depletion medium from the downstream surface through the upstream surface; and, (c) passing the wash fluid through the leukocyte depletion medium from the upstream surface through the downstream surface via gravity.

In some embodiments of the methods, (b) and (c) are repeated at least once with additional wash fluid.

In some embodiments, the biological fluid or cryoprotectant fluid is passed through the porous leukocyte depletion medium via gravity or vacuum. Passing the wash fluid through the leukocyte depletion medium from the downstream surface through the upstream surface comprises creating a negative pressure (vacuum) upstream of the upstream surface, or comprises creating a positive pressure downstream of the downstream surface.

Advantageously, leukocytes can be obtained while reducing the presence of red blood cells and/or platelets. Minimizing the presence of red blood cells and platelets is particularly advantageous for cell therapy applications. Alternatively, or additionally, minimizing the presence of DMSO is particularly advantageous for cell therapy applications, especially for those involving neonatals, infants, and children.

Some embodiments of the methods further comprise eluting leukocytes from the leukocyte depletion medium by passing an elution fluid through the leukocyte depletion medium from the downstream surface through the upstream surface, and recovering the eluted leukocytes, or lysing the leukocytes while retained by the leukocyte depletion medium. In one embodiment including lysing the leukocytes, the method further comprises analyzing the content of the lysed leukocytes, e.g., comprising analyzing nucleic acids from the lysed leukocytes.

In one preferred embodiment of the method, the eluted leukocytes are administered to a subject, such as a patient.

If desired, embodiments of the method can be carried out using multiwell device, or individual filter devices. For example, using a multiwell device having a plurality of wells having leukocyte depletion media therein, separate portions of biological fluid and wash fluid are passed through the leukocyte depletion media in separate wells.

The invention can be carried out using biological fluid from a variety of sources, particularly mammals (the cells in the cryoprotectant fluid can also be from a variety of sources). It is preferred that the mammals are from the order Carnivora, including Felines (cats) and Canines (dogs), the order Artiodactyla, including Bovines (cows) and Swines (pigs) or of the order Perssodactyla, including Equines (horses). Typically, the mammals are of the order Primates, Ceboids, or Simoids (monkeys) or of the order Anthropoids (humans and apes). An especially preferred mammal is the human.

In accordance with embodiments of the invention, any suitable volume of biological fluid or cryoprotectant fluid can be processed, and a variety of leukocyte depletion media and leukocyte depletion filter configurations are suitable, e.g., media or filters having diameters in the range from, for example, about 0.3 inches (about 7 mm) or less, to about 5 inches (about 12 cm), or more.

The cord blood concentrates and stem cell concentrates used in accordance with the invention can be produced (e.g., to obtain an enriched white cell fraction, combined with a cryoprotectant agent, frozen, and thawed) by a variety of techniques known in the art before the leukocytes are purified (and, preferably, eluted) in accordance with embodiments of the invention.

Each of the components of the invention will now be described in more detail below, wherein like components have like reference numbers.

A variety of porous leukocyte depletion media are suitable for use in the invention. Suitable leukocyte depletion media include commercially available media, for example, media available from Pall Corporation (Port Washington, NY).

A variety of wash fluids are suitable for use in the invention. Typically, the wash fluid is physiologically compatible with the desired cells, and does not substantially effect the cells. Illustrative fluids include, for example, saline, for example, phosphate buffered saline (PBS).

Washing can be accomplished at any suitable fluid flow rate. Typically, passing the wash fluid through the leukocyte depletion medium from the downstream surface through the upstream surface comprises creating a negative pressure upstream of the upstream surface, e.g., by withdrawing the plunger in a syringe barrel, wherein the syringe barrel is connected to the inlet of the filter device, or creating a vacuum in the well(s) of a multiple well device. Preferably, passing the wash fluid through the leukocyte depletion medium from the upstream surface through the downstream surface is accomplished via gravity.

In those embodiments including elution, the desired retained cells, i.e., leukocytes (and in some embodiments, stem cells) retained (e.g., captured and/or adsorbed) by the filter are released by backflushing from the porous leukocyte depletion medium, i.e., by passing the elution fluid through the porous medium in a direction from the downstream side towards the upstream side, and through the inlet port, such that the elution fluid containing the cells is passed from the inlet port into a cell collection container communicating with the inlet port.

The backflushing can be accomplished at any suitable fluid flow rate, e.g., about 0.1-15 L/min/m², although flow rates significantly more or less than this range can be used. For example, backflushing can be accomplished at a fluid flow rate of about 0.5-10 L/min/m², such as about 1-8 L/min/m²; more preferably the flow rate is about 1.5-7 L/min/m², such as about 2-6 L/min/m² or even about 2.5-5 L/min/m² (e.g., about 3-4 L ml/min/m²). The most preferable flow rate may depend upon the viscosity and/or the temperature of the elution fluid, and the nature of the filter medium. Thus, in some applications, such as when more gentle treatment is desired, backflushing can be accomplished at a flow rate about 1-100 ml/min/m², (e.g., about 15-85 ml/min/m²); more preferably the flow rate is about 30-70 ml/min/m² or even about 40-60 ml/min/m² (e.g., about 50 ml/min/m²)_{.}

A variety of elution fluids are suitable for use in the invention. Typically, the fluid is physiologically compatible with the desired cells, and does not substantially effect the cells. Illustrative fluids include, for example, saline, as well as those fluids, including more viscous fluids, disclosed in U.S. Patents 6,544,751 and 7,291,450.

In those embodiments wherein leukocytes are purified in accordance with the invention, and are subsequently lysed in or on the leukocyte depletion medium (rather than eluted from the medium), a variety of reagents and protocols are suitable for treating and lysing the leukocytes, and purifying and/or isolating the nucleic acids (preferably, RNA, but embodiments of the invention include purifying and/or isolating DNA) released from the leukocytes and are known in the art. If desired, the leukocytes can be treated to stabilize the cells before lysing. The stabilized cells can be maintained in or on the leukocyte depletion medium for a suitable period before lysis, or the cells can be lysed shortly or immediately after stabilization. Preferably, the leukocytes are disputed using a solution such that RNA is rapidly released while inactivating nucleases. Illustrative reagents and protocols, including purifying RNA, isolating total RNA, and depleting globin RNA from purified RNA, are disclosed in, for example, "LeukoLOCK™ Total RNA Isolation System, *Globin mRNA-Depleted Total RNA from Whole Blood Samples,"* Instruction Manual, Catalog #AM 1923, AM1933, AM1934, (Manual 1923M Revision B) Ambion Inc., Austin, TX (2007).

A variety of containers and devices comprising containers are suitable for use in the invention, wherein the containers can be arranged upstream and/or downstream of the leukocyte depletion media. Suitable containers are known in the art. Typically, one or more of the containers are physiologically compatible with the biological fluid, the desired cells, the wash fluids, and/or the elution fluids, and do not substantially effect the cells. Since a plurality of containers can be used in embodiments of the invention, it is not a requirement that a single container be physiologically compatible with the biological fluid, the desired cells, the wash fluids, and the elution fluids. For example, one container can be used for passing biological fluid or cryoprotectant fluid through the leukocyte depletion medium (and thus need not be physiologically compatible with wash fluid and/or elution fluid), another can be used for receiving wash fluid through the leukocyte depletion medium and passing wash fluid through the leukocyte depletion medium, and another can be used for receiving eluted cells and elution fluid.

In some embodiments, the container(s) used for passing biological fluid or cryoprotectant fluid through the leukocyte depletion medium, and receiving wash fluid through the leukocyte depletion medium and passing wash fluid through the leukocyte depletion medium, is/are substantially non-flexible, e.g., non-flexible containers such as syringe barrels. However, embodiments of the invention are not so limited. For example, the container(s) used for passing biological fluid or cryoprotectant fluid through the leukocyte depletion medium, and receiving wash fluid through the leukocyte depletion medium and passing wash fluid through the leukocyte depletion medium can comprise flexible containers such as blood bags (e.g., plasticized blood bags), having flexible side walls, typically, wherein the container(s) has/have at least two ports, e.g., an inlet port and an outlet port. The flexible side walls can be compressed or a vacuum can be created such that air and/or liquid passes from the container and/or the flexible side walls expand when air and/or liquid enters the bag and the bag is pressurized, and the compliant container directs the air and/or liquid out when the outlet port is opened.

Embodiments of the invention are suitable for use in open systems, and embodiments of the invention can be carried out while maintaining closed systems.

The following definitions are used in accordance with the invention.

Biological Fluid. A biological fluid includes any treated or untreated fluid associated with living organisms, particularly blood, including whole blood, warm or cold blood, cord blood, and stored or fresh blood; treated blood, such as blood diluted with at least one physiological solution, including but not limited to saline, nutrient, and/or anticoagulant solutions; blood components, such as platelet concentrate (PC), platelet-rich plasma (PRP), platelet-poor plasma (PPP), platelet-free plasma, plasma, fresh frozen plasma (FFP), components obtained from plasma, packed red cells (PRC), transition zone material or buffy coat (BC); blood products derived from blood or a blood component or derived from bone marrow; stem cells; cord blood; red cells separated from plasma and resuspended in a physiological solution or a cryoprotective fluid; and platelets separated from plasma and resuspended in a physiological solution or a cryoprotective fluid. A biological fluid also includes a physiological solution comprising a bone marrow aspirate. The biological fluid may have been treated to remove some of the leukocytes before being processed according to the invention. As used herein, blood product or biological fluid refers to the components described above, and to similar blood products or biological fluids obtained by other means and with similar properties.

A "cryoprotectant fluid" can include, for example, a cryoprotectant agent and mixtures thereof, such as, but not limited to dimethyl sulfoxide (DMSO), glycerol, polyvinylpyrrolidone, polyethylene glycol, albumin, dextran, sucrose, ethylene glycol, I-erythritol, D-ribitol, D-mannitol, D-sorbitol, I-inositol, D-lactose, choline chloride, amino acids, methanol, acetamide, glycerol monoacetate, and inorganic salts. Exemplary treatment solutions include those disclosed in International Publication No. WO 96/17514. Typically, embodiments, DMSO is used, which is nontoxic to cells in low concentration. It is believed that, being a small molecule, DMSO freely permeates the cell and protects intracellular organelles by combining with water to modify its freezability and prevent damage from ice formation. The addition of plasma (e.g., to a concentration of about 20-25%) can augment the protective effect of DMSO.

A "unit" is the quantity of biological fluid from a donor or derived from one unit of whole blood. It may also refer to the quantity drawn during a single donation. Typically, the volume of a unit varies, the amount differing from patient to patient and from donation to donation. Multiple units of some blood components, particularly platelets and buffy coat, may be pooled or combined, typically by combining four or more units.

As used herein, the term "closed" refers to a system that allows the collection and processing (and, if desired, the manipulation, e.g., separation of portions, separation into components, filtration, storage, and preservation) of cryoprotectant fluid or biological fluid, e.g., donor blood, blood samples, and/or blood components, without the need to compromise the sterile integrity of the system. A closed system can be as originally made, or result from the connection of system components using what are known as "sterile docking" devices. Illustrative sterile docking devices are disclosed in, for example, U.S. Patents 4,507,119, 4,737,214, and 4,913,756.

A variety of materials can be used, including synthetic polymeric materials, to produce the porous leukocyte depletion media (preferably, fibrous porous leukocyte depletion media) of the filter elements according to the invention. Suitable synthetic polymeric materials include, for example, polybutylene terephthalate (PBT), polyethylene, polyethylene terephthalate (PET), polypropylene, polymethylpentene, polyvinylidene fluoride, polysulfone, polyethersulfone, nylon 6, nylon 66, nylon 6T, nylon 612, nylon 11, and nylon 6 copolymers, wherein polyesters, e.g., PBT and PET, are more preferred. Typically, the fibrous porous media are prepared from melt-blown fibers. For example, U.S. Patents 4,880,548; 4,925,572, 5,152,905, and 6,074,869, disclose porous leukocyte depletion filters and filter elements prepared from melt-blown fibers.

A leukocyte depletion filter element can have any suitable pore structure, e.g., a pore size (for example, as evidenced by bubble point, or by K_{L} as described in, for example, U.S. Patent 4,340,479, or evidenced by capillary condensation flow porometry), a pore rating, a pore diameter (e.g., when characterized using the modified OSU F2 test as described in, for example, U.S. Patent 4,925,572), or removal rating that reduces or allows the passage therethrough of one or more materials of interest as the fluid is passed through the element. While it is believed leukocytes are primarily removed by adsorption, they can also be removed by filtration. The pore structure can be selected to remove at least some level of leukocytes, while allowing the passing therethrough of other components, e.g., plasma, platelets, and red blood cells. The pore structure used depends on the composition of the fluid to be treated, and the desired effluent level of the treated fluid.

The filter element can have any desired critical wetting surface tension (CWST, as defined in, for example, U.S. Patent 4,925,572). The CWST can be selected as is known in the art, e.g., as additionally disclosed in, for example, U.S. Patents 5,152,905, 5,443,743, 5,472,621, and 6,074,869. Typically, the filter element has a CWST of greater than about 53 dynes/cm (about 53 x 10⁻⁵ N/cm), more typically greater than about 58 dynes/cm (about 58 x 10⁻⁵ N/cm), and can have a CWST of about 66 dynes/cm (about 66 x 10⁻⁵ N/cm) or more. In some embodiments, the element has a CWST of 75 dynes/cm (about 75 x 10⁻⁵ N/cm) or more, e.g., in the range of about 80 to about 100 dynes/cm (about 80 to about 100 x 10⁻⁵ N/cm).

The surface characteristics of the element can be modified (e.g., to affect the CWST, to include a surface charge, e.g., a positive or negative charge, and/or to alter the polarity or hydrophilicity of the surface) by wet or dry oxidation, by coating or depositing a polymer on the surface, or by a grafting reaction. Modifications include, e.g., irradiation, a polar or charged monomer, coating and/or curing the surface with a charged polymer, and carrying out chemical modification to attach functional groups on the surface. Grafting reactions may be activated by exposure to an energy source such as gas plasma, vapor plasma, corona discharge, heat, a Van der Graff generator, ultraviolet light, electron beam, or to various other forms of radiation, or by surface etching or deposition using a plasma treatment.

The filter and/or filter device can include additional elements, layers, or components, that can have different structures and/or functions, e.g., at least one of prefiltration, support, drainage, spacing and cushioning.

The filter element, in some embodiments a filter comprising a plurality of filter elements, can be disposed in a housing comprising at least one inlet and at least one outlet and defining at least one fluid flow path between the inlet and the outlet, wherein the filter element is across the fluid flow path, to provide a filter device. Typically, the filter device is sterilizable. Any housing of suitable shape and providing at least one inlet and at least one outlet may be employed.

Alternatively, filter elements or filters comprising one or more filter elements can be disposed in multiple well devices or filter plates, wherein filter elements or filters comprising a plurality of filter devices are disposed in the wells. Suitable devices and filter plates are known in the art and are commercially available, e.g., from Pall Corporation (Port Washington, NY) under the tradenames ACROWELL^{™} and ACROPREP^{™} and/or described in, for example, International Publication No. WO 2002/096563.

The housing can be fabricated from any suitable rigid impervious material, including any impervious thermoplastic material, which is compatible with the biological fluid being processed. Typically, the housing is fabricated from a polymer. In some embodiments, the polymer a transparent or translucent polymer, such as an acrylic, polypropylene, polystyrene, or a polycarbonated resin. Such a housing is easily and economically fabricated, and allows observation of the passage of fluid through the housing.

If desired, the housing can be sealed as is known in the art, utilizing, for example, an adhesive, a solvent, laser welding, radio frequency sealing, ultrasonic sealing and/or heat sealing. Additionally, or alternatively, the housing can be sealed via injection molding.

According to one embodiment of the present invention, the method for purifying leukocytes may be carried out as follows:

1. An empty syringe barrel, connected to the inlet of a syringe filter including a leukocyte depletion medium, is arranged such that the outlet of the syringe filter is in fluid communication with a receiving container. Leukocyte-containing biological fluid is placed in the barrel, and drains through the filter via gravity, wherein leukocytes are captured by the leukocyte depletion medium.

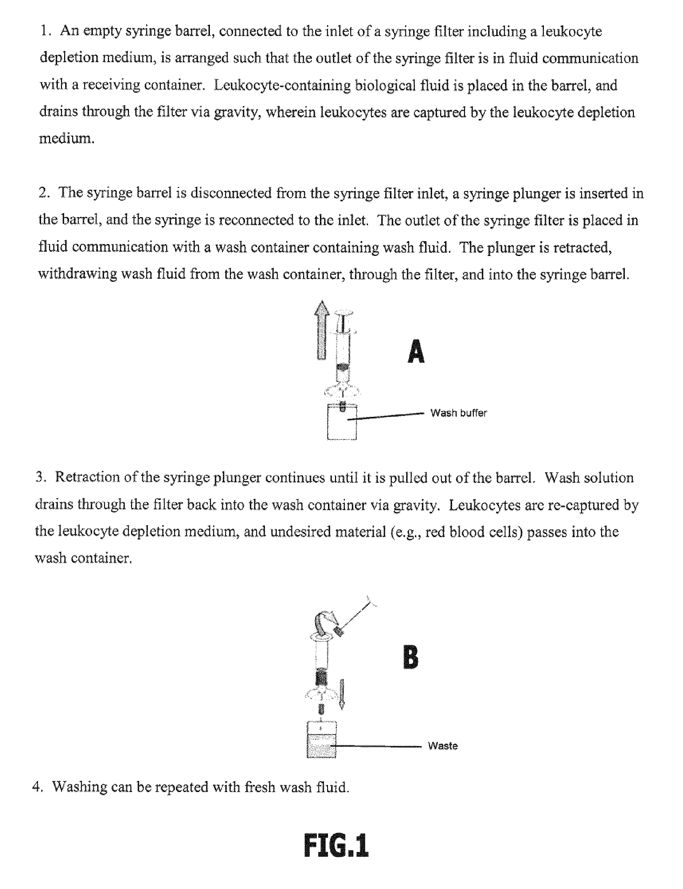

2. The syringe barrel is disconnected from the syringe filter inlet, a syringe plunger is inserted in the barrel, and the syringe is reconnected to the inlet. The outlet of the syringe filter is placed in fluid communication with a wash container containing wash fluid. The plunger is retracted, withdrawing wash fluid from the wash container, through the filter, and into the syringe barrel (cf. part A of Figure 1).

3. Retraction of the syringe plunger continues until it is pulled out of the barrel. Wash solution drains through the filter back into the wash container via gravity. Leukocytes are re-captured by the leukocyte depletion medium, and undesired material (e.g., red blood cells) passes into the wash container (cf. part B of Figure 1).

4. Washing can be repeated with fresh wash fluid.

The following examples further illustrate the invention but, of course, should not be construed as in any way limiting its scope.

### EXAMPLE 1

This example demonstrates leukocytes can be purified and recovered with a good yield and while minimizing red blood cell contamination according to an embodiment of the invention.

The filter devices are 25 mm ACRODISC® syringe filters with Leukosorb leukocyte depletion media (Pall Corporation, Port Washington, NY). The inlet ports of the devices are connected to empty 5 mL syringe barrels, the outlet ports are connected to tubing leading to a waste container, and 9 mL samples of freshly collected blood from donors are treated with EDTA or CP2D anticoagulant, drain, via gravity, through the filters. The filtered blood is discarded.

New 5 mL syringe barrels (including plungers) are connected to the inlet ports of the devices, and the tubing connected to the outlet ports of the devices are placed in containers containing about 8 µL of phosphate buffered saline (PBS) ("the waste containers"). As shown in Figure 1, the syringe plungers are withdrawn to pull PBS from the waste containers, through the downstream surfaces of the leukocyte depletion media and the upstream surfaces, and into the syringe barrels. As shown in Figure 1, after the PBS is pulled into the syringe barrels, the syringe plungers are pulled out of the barrels, while the device outlets remain in contact with the PBS in the waste containers. The PBS in each syringe barrel then drains, via gravity through the upstream surface and the downstream surface, and into the waste container. The wash is repeated, as another volume of PBS is pulled through the media and allowed to drain as described above.

5 mL of elution solution comprising 6% dextran 70 in PBS as generally described in U.S. Patent 6,544,751 is passed from the outlet of each device through the inlet, and the eluted leukocytes are recovered.

Influent, effluent, and eluted leukocyte and red blood cell counts are taken. The results are as follows:

**Table 1**

| Sample | Anticoagulant | Trapped WBCs, % to Control | Recovered WBCs, % to Control | Residual RBCs in the WBCs eluate, % to Control |
|---|---|---|---|---|
| 1 | EDTA | 89 | 61 | 0.1 |
| 2 | EDTA | 96 | 77 | 0.03 |
| 3 | CP2D | 88 | 62 | 0.01 |
| 4 | CP2D | 96 | 84 | 0.02 |
| 5 | EDTA | 86 | 71 | 0.08 |
| 6 | CP2D | 96 | 70 | 0.05 |
| 7 | EDTA | 90 | 80 | 0.1 |
| 8 | CP2D | 89 | 81 | 0.03 |
| 9 | EDTA | 87 | 63 | 0.1 |

In each sample, compared to the controls, over 85% of the white blood cells are trapped and over 60% of the white blood cells compared to the control are recovered, and, compared to the controls, 1% or less red blood cells are present in the eluted while blood cells.

### EXAMPLE 2

This example demonstrates the reduction in red blood cell contamination while purifying and recovering leukocytes according to an embodiment of the invention.

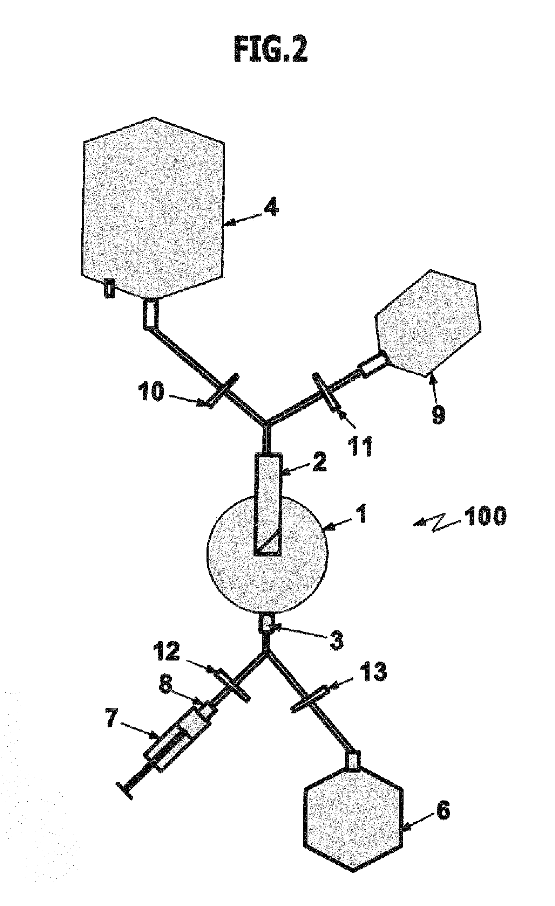

The system 100 used for carrying out this Example is shown in Figure 2, showing a filter device 1, having an inlet port 2 and an outlet port 3, source bag (blood bag) 4, waste bag 6, leukocyte sampling bag 9, syringe 7, injection port 8, and clamps 10-13.

The filter devices are PURECELL® PXLA High Efficiency Leukocyte Reduction Filters for Apheresis Platelet Transfusion with leukocyte depletion media (Pall Corporation, Port Washington, NY). The inlet port 2 of the filter device 1 is connected through tubing and a "Y" connector to the blood bag 4 and to the leukocyte sampling bag 9, the device outlet port 3 is connected to tubing leading through another "Y" connector to the waste bag 6 and to the injection port 8, which is connected to syringe 7.

The syringe is filled with elution solution and attached to port 8 while clamp 12 is closed.

In order to control the flow, clamps are placed on the tubing between the inlet port and the blood bag and the sampling bag (clamps 10 and 11), and between the outlet port and the waste bag and the injection port (clamps 12 and 13). All the clamps are initially closed.

The blood bag contains 50 mL of blood collected from donors and mixed with anticoagulant according to a standard blood bank procedure. The system is arranged vertically, and clamps 10 and 13 are opened, blood is filtered via gravity, passing into waste container 6, and leucocytes are captured on the leukocyte depletion medium in the filter device 1.

Clamp 13 is closed, clamp 12 is opened and 20 mL of PBS wash solution is injected via syringe 7 through injection port 8, such that the wash solution passes through the downstream surface of the leukodepletion medium and through upstream surface and into the blood bag 4. Clamps 10 and 13 are opened again and wash solution is allowed to drain via gravity through the filter device into the waste bag 6.

The wash procedure is repeated once more with fresh PBS.

The syringe 7 is filled with elution solution (6% dextran 40 in PBS) and connected to the injection port 8.

Clamps 10 and 13 are closed, and clamps 11 and 12 are opened, and 24 uL of the elution solution is injected from the syringe 7 into the injection port 8 such that leukocytes are eluted and pass, with the elution solution, into the sampling bag 9.

For one experiment, the wash fluid is not drained into a waste bag, for the other experiment, the wash fluid is drained into waste bag 6. Additionally, leukocytes are recovered without the use of a wash, and the results for the various filtrations are compared.

The results are as follows (percent recovery into elution buffer):

| wash | WBC | lymphocytes | monocytes | granulocytes | RBC | platelets |
|---|---|---|---|---|---|---|
| none | 59 | 75 | 77 | 50 | 15 | 25 |
| 2x | 65 | 77 | 86 | 56 | 3.6 | 18 |
| 2x drain into waste bag | 62 | 70 | 80 | 56 | 3.4 | 19 |

The results show using the wash reduced red blood cell contamination by over 66% as compared to no wash.

### EXAMPLE 3

This example demonstrates that leukocytes can be purified and lysed and the leukocyte nucleic acids can be analyzed according to an embodiment of the invention.

The filter devices are 25 mm Acrodisc® syringe filters with Leukosorb leukocyte depletion media (Pall Corporation, Port Washington, NY).

9 mL samples of freshly collected blood from donors are treated with EDTA anticoagulant, and processed as follows. Influent and effluent leukocyte counts are taken.

For the first experiment, the inlet ports of the devices are connected to empty 5 mL syringe barrels, the outlet ports are connected to tubing leading to a waste container, and 9 mL samples of freshly collected blood from donors were treated with EDTA anticoagulant, drain, via gravity, through the filters. The filtered blood is discarded.

New 5 mL syringe barrels (including plungers) are connected to the inlet ports of the devices, and the tubing connected to the outlet ports of the devices are placed in waste containers containing about 8 µL of phosphate buffered saline (PBS). The syringe plungers are withdrawn to pull PBS from the containers, through the downstream surfaces of the leukocyte depletion media and the upstream surfaces, and into the syringe barrel. After the PBS is pulled into the syringe barrels, the syringe plungers are pulled out of the barrels, while the device outlets remain in contact with the PBS in the waste containers. The PBS in each syringe barrel then drains, via gravity through the upstream surface and the downstream surface, and into a waste container. The wash is repeated, as another volume of PBS is pulled through the media and allowed to drain as described above.

2.5 mL of pH-adjusted Lysis/Binding Solution is passed through the medium using a syringe, and collected in a lysate collection tube. Lysate is added with 2.5 mL of Nuclease-free Water, and Proteinase K. Total RNA is isolated as described in "LeukoLOCK™ Total RNA Isolation System, *Globin mRNA-Depleted Total RNA from Whole Blood Samples,"* Instruction Manual, Catalog #AM 1923, AM1933, AM1934, (Manual 1923M Revision B) Ambion Inc., Austin, TX (2007) (the "LeukoLOCK™ Total RNA Isolation System Manual").

In the second experiment, the blood is passed through the filter following the procedure as described in the LeukoLOCK™ Total RNA Isolation System Manual, and the leukocytes are isolated, stabilized, lysed, and the total RNA is isolated as described in the LeukoLOCK™ Total RNA Isolation System Manual.

The efficiency of leukocyte capture, the RNA yield, and the optical density ratio of absorbance at 260 and 280 nm (A_{260/280}) is determined in each experiment.

The results are as follows:

| Experiment | Efficiency of WBCs capture on the filter, % | RNA yield, ug/blood sample | A_{260/280} |
|---|---|---|---|
| First (embodiment of the invention) | 96 | 12 | 2.03 |
| Second | 74 | 8.9 | 2.10 |

This example demonstrates the improvement in leukocyte purification and total RNA yield according to an embodiment of the invention.

### EXAMPLE 4

This example demonstrates that leukocytes can be purified and lysed and the leukocyte genomic DNA can be analyzed according to an embodiment of the invention.

Leukocytes are purified and recovered as described in Example 1.

200 µL aliquots of eluted leukocytes are processed to isolate genomic DNA using phenol/chloroform extraction as is known in the art. The isolated DNA is analyzed via UV-spectrophotometry, agarose gel electrophoresis, and PCR.

The results are as follows (wherein K=1000):

| WBC Sample | WBC concentration K/µL | DNA yield µg/sample | OD 260/280 |
|---|---|---|---|
| 1 | 2.95 | 2.9 | 1.94 |
| 2 | 2.65 | 2.5 | 1.98 |
| 3 | 3.00 | 3.1 | 1.82 |

Agarose gel electrophoresis of the obtained DNA shows high molecular weight DNA is isolated from the recovered leukocytes. The DNA is intact and amplifiable, shown by amplification using PCR and amelogenin primers.

### EXAMPLE 5

This example demonstrates that leukocytes can be purified and lysed and the leukocyte nucleic acids can be analyzed according to an embodiment of the invention, using varied layers of leukocyte depletion media.

The filter devices are 25 mm Acrodisc® syringe filters with 4 or 8 layers of Leukosorb leukocyte depletion media (Pall Corporation, Port Washington, NY).

9 mL samples of freshly collected blood from donors are treated with EDTA anticoagulant, withdrawn into 10 µL syringes, and processed as follows. Influent and effluent leukocyte counts are taken.

The inlet ports of the devices are connected to the filled syringes, the outlet ports are connected to tubing leading to a waste container, the syringe plungers are pressed, and samples of blood are passed at a flow rate of 3-5 drops per second through the filters. The filtered blood is discarded.

New 5 mL syringe barrels (including plungers) are connected to the inlet ports of the devices, and the tubing connected to the outlet ports of the devices are placed in waste containers containing about 8 µL of phosphate buffered saline (PBS). The syringe plungers are withdrawn to pull PBS from the containers, through the downstream surfaces of the leukocyte depletion media and the upstream surfaces, and into the syringe barrels. After the PBS is pulled into the syringe barrels, the syringe plungers are pulled out of the barrels, while the device outlets remain in contact with the PBS in the waste containers. The PBS in each syringe barrel then drains, via gravity through the upstream surface and the downstream surface, and into a waste container. The wash is repeated, as another volume of PBS is pulled through the media and allowed to drain as described above.

2.5 mL of pH-adjusted Lysis/Binding Solution is passed through the medium using a syringe, and collected in a lysate collection tube. Lysate is added with 2.5 mL of Nuclease-free Water, and Proteinase K. Total RNA is isolated as described in "LeukoLOCK™ Total RNA Isolation System, *Globin mRNA-Depleted Total RNA from Whole Blood Samples,"* Instruction Manual, Catalog #AM 1923, AM1933, AM1934, (Manual 1923M Revision B) Ambion Inc., Austin, TX (2007) (the "LeukoLOCK™ Total RNA Isolation System Manual").

The efficiency of leukocyte capture, the RNA yield, and the optical density ratio of absorbance at 260 and 280 nm (A_{260/280}) is determined.

The results are as follows:

| Filter device | Efficiency of WBCs capture on the filter, % | RNA yield, ug/blood sample | A_{260/280} |
|---|---|---|---|
| 4 layers | 67.2 | 9.5 | 2.06 |
| 8 layers | 99.5 | 13.6 | 2.11 |

### EXAMPLE 6

This example demonstrates the reduction in red blood cell and platelet contamination while purifying and recovering leukocytes according to an embodiment of the invention.

The system used for carrying out this Example is shown in Figure 2.

The filter devices are PURECELL® PXLA High Efficiency Leukocyte Reduction Filters for Apheresis Platelet Transfusion with leukocyte depletion media (Pall Corporation, Port Washington, NY). The inlet port 2 of the filter device 1 is connected through tubing and a "Y" connector to the blood bag 4 and to the leukocyte sampling bag 9, the device outlet port 3 is connected to tubing leading through another "Y" connector to the waste bag 6 and to the injection port 8, which is connected to syringe 7. The system is arranged horizontally on a bench-top, with the waste bag hanging from the side of the bench.

The source bag, containing 50 mL of whole blood mixed with anticoagulant and 10 mL of air is placed on a box so that the bag is 2 inches above the filter on the bench-top. The waste bag is hung below the bench-top to prevent reverse flow. The clamps are opened allowing the blood to pass from the source bag through the filter and into the waste bag. The tubing below the filter is clamped, and the filter backwashed via the injection port using PBS or PBS/dextran solutions 6% dextran 70 in PBS, in 20 mL increments for a total wash volume of 20, 40, or 60 mL, or in single 40 mL or 60 mL increments, and the wash fluid passes into the source bag. For the control, the filter is not backwashed.

The wash fluid and cells are mixed in the source bag and the system is again placed on the bench-top so that the source bag is 2 inches above the filter and the wash bag hangs from the side of the bench. The mixture passes from the source bag through the filter and into the waste bag.

The wash procedure is repeated until the specific wash volume is achieved.

The results are analyzed. No backwash yields red blood cell contamination of about 14%, a 20 mL PBS backwash reduces red blood cell contamination to about 6.5%, a 2 x 20 mL PBS backwash reduces it to about 3.4%, and a 3 x 20 mL PBS backwash reduces it to about 1.8%.

The leukocyte recovery without a backwash is about 61%, about 66% for 20 mL and 2 x 20 mL PBS backwashes, and about 63% for a 3 x 20 mL PBS backwash.

No backwash yields platelet contamination of about 19%, as does a 20 mL PBS backwash. A 2 x 20 mL PBS backwash reduces platelet contamination to about 13%, and a 3 x 20 mL PBS backwash reduces it to about 6%.

With respect to PBS/dextran, no backwash yields red blood cell contamination of about 14%, about 69% leukocyte recovery, and about 22% platelet contamination.

The use of PBS/dextran reduces red blood cell contamination (about 8% 20 mL PBS/dextran; about 3% for 2 x 20 mL PBS/dextran), and, platelet contamination (about 9% 20 mL PBS/dextran and 2 x 20 mL PBS/dextran). However, the use of PBS/dextran reduces leukocyte recovery compared to no backwash (about 69% no backwash, 66% 20 mL PBS/dextran; about 56% for 2 x 20 mL PBS/dextran).

In another experiment, comparing the results of a control, and PBS backwashes of 2 x 20 mL, 40 mL single backwash, and 60 mL single backwash, the results are generally similar for the 3 backwashes (all showing increased leukocyte recovery, and lower red blood cell and platelet contamination compared to the control), although the 2 x 20 mL backwash yields about 18% red blood cell contamination, and the other backwashes yield about 8% red blood cell contamination (compared to about 22% for the control).

### EXAMPLE 7

This example demonstrates the reduction in DMSO concentration while recovering leukocytes according to an embodiment of the invention.

Blood is collected from a donor into VACUTAINER® tubes (Becton Dickenson, Franklin Lakes, NJ) containing EDTA anticoagulant. The blood is processed to obtain and isolate buffy coat.

A cryoprotectant fluid containing 10% DMSO and 90% same donor plasma is prepared, chilled, and mixed with the buffy coat. Two 6 mL samples of buffy coat mixed with cryoprotectant fluid are obtained.

The samples are filtered, and the leukocytes are washed and eluted as generally described in Example 1, with the exception that the leukocytes in this Example are eluted with PBS, rather than an elution solution including dextran.

Influent, effluent, and eluted leukocyte cell counts are taken. Additionally, the initial total protein concentration is determined, as is the total protein concentration in the eluted leukocyte-containing fluid, as the change in protein concentration correlates with the reduction of DMSO concentration. The results are as follows:

| sample | WBC recovery % | Residual DMSO concentration % |
|---|---|---|
| 1 | 65 | 0.11 |
| 2 | 62 | 0.14 |

The results show that DMSO concentration can be decreased by about 90% while recovering leukocytes.

### EXAMPLE 8

This example demonstrates leukocytes can be purified and recovered while minimizing platelet contamination according to an embodiment of the invention.

Blood samples are filtered and leukocytes are washed and eluted using devices as generally described in Example 1.

Influent, effluent, and eluted leukocyte and platelet counts are taken. The results are as follows (wherein K=1000).

| Sample ID | Platelet count influent, K/µL | Platelet count effluent, K/µL | Platelet count in eluted WBCs, K/µL | Reduction in platelets on the filter, % | Residual Platelets in eluted WBC, % | WBC recovery in eluate % |
|---|---|---|---|---|---|---|
| **Blood with EDTA anticoagulant** | | | | | | |
| 1 | 380 | 321 | 14 | 85 | 4 | 62 |
| 2 | 380 | 323 | 15 | 79 | 4 | 75 |
| 3 | 380 | 301 | 17 | 62 | 11 | 86 |
| 4 | 190 | 118 | 15 | 59 | 8 | 88 |
| 5 | 190 | 112 | 20 | 85 | 4 | 84 |

| **Blood with CP2D anticoagulant** | | | | | | |
|---|---|---|---|---|---|---|
| 1 | 335 | 21 | 4 | 6 | 1 | 63 |
| 2 | 335 | 168 | 24 | 50 | 7 | 84 |
| 3 | 335 | 157 | 17 | 47 | 5 | 80 |
| 4 | 175 | 34 | 7 | 19 | 4 | 74 |
| 5 | 175 | 49 | 9 | 28 | 5 | 78 |
| 6 | 175 | 60 | 8 | 34 | 5 | 72 |

### EXAMPLE 9

This example demonstrates the reduction in red blood cell contamination while purifying and recovering leukocytes and maintaining a closed system in according to an embodiment of the invention.

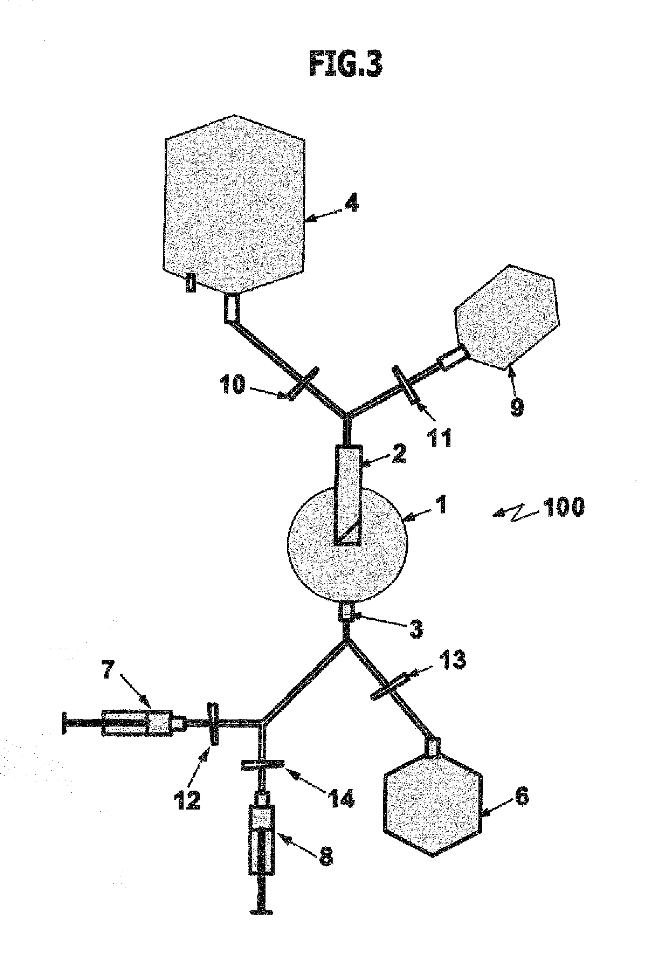

The system 100 used for carrying out this Example is shown in Figure 3, showing a filter device 1, having an inlet port 2 and an outlet port 3, source bag (blood bag) 4, waste bag 6, leukocyte sampling bag 9, syringe 7 containing wash solution, syringe 8 containing elution solution, and clamps 10-14.

The filter devices are PURECELL® PXLA High Efficiency Leukocyte Reduction Filters for Apheresis Platelet Transfusion with leukocyte depletion media (Pall Corporation, Port Washington, NY). The inlet port 2 of the filter device 1 is connected through tubing and a "Y" connector to the blood bag 4 and to the leukocyte sampling bag 9, the device outlet port 3 is connected via a "Y" connector to tubing leading to the waste bag 6, and to another "Y" connector leading to syringes 7 and 8.

In order to control the flow, clamps 10-14 are placed on the tubing between the inlet port and the blood bag and the sampling bag (clamps 10 and 11), and between the outlet port and the waste bag and the wash and the elution syringes (clamps 12-14). All clamps are initially closed.

The blood bag contains 50 mL of blood collected from donors and mixed with anticoagulant according to a standard blood bank procedure. Clamps 10 and 13 are opened, blood is filtered via gravity, passing into waste bag 6 and leucocytes are captured on the leukocyte depletion medium in the filter device 1.

Clamp 13 is closed, the clamp 12 is opened and 20 mL of PBS is injected via syringe 7 such that the wash solution passes through the downstream surface of the leukocyte depletion medium and through the upstream surface and into blood bag 4. Clamp 12 is closed and clamp 13 is opened again and wash solution is allowed to drain via gravity through the filter device into the waste bag 6.

The wash procedure is repeated once more with fresh PBS. The wash fluid is drained into a waste bag 6.

Clamps 10 and 13 are closed. Clamps 11 and 14 are opened and 24 µL of the elution solution is injected from the syringe 8 such that leukocytes are eluted and pass, with elution solution, into the sampling bag 9.

The results show, similar to those in Example 2, using the wash reduced red blood cell contamination as compared to no wash. Additionally, this Example shows an embodiment of the method can be carried out while maintaining a closed system.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. A method for purifying leukocytes, the method comprising
(a) passing a biological fluid comprising leukocytes, or a cryoprotectant fluid comprising leukocytes, through a porous leukocyte depletion medium having an upstream surface and a downstream surface, the fluid passing from the upstream surface through the downstream surface, wherein leukocytes are retained by the leukocyte depletion medium;
(b) passing a wash fluid through the leukocyte depletion medium from the downstream surface through the upstream surface; and,
(c) passing the wash fluid through the leukocyte depletion medium from the upstream surface through the downstream surface via gravity.

2. The method of claim 1, comprising passing the biological fluid or the cryoprotectant fluid through the porous leukocyte depletion medium via gravity

3. The method of claim 1, comprising passing the biological fluid or the cryoprotectant fluid through the porous leukocyte depletion medium via vacuum.

4. The method of any one of claims 1-3, further comprising repeating (b) and (c) at least once with additional wash fluid.

5. The method of any one of claims 1-4, wherein the cryoprotectant fluid comprises DMSO.

6. The method of any one of claims 1-5, wherein passing the wash fluid through the leukocyte depletion medium from the downstream surface through the upstream surface comprises creating a vacuum upstream of the upstream surface.

7. The method of any one of claims 1-5, wherein passing the wash fluid through the leukocyte depletion medium from the downstream surface through the upstream surface comprises creating a positive pressure downstream of the downstream surface.

8. The method of any one of claims 1-7, further comprising eluting leukocytes from the leukocyte depletion medium by passing an elution fluid through the leukocyte depletion medium from the downstream surface through the upstream surface, and recovering the eluted leukocytes.

9. The method of any one of claims 1-7, further comprising lysing the retained leukocytes.

10. The method of claim 9, further comprising processing nucleic acids from the lysed leukocytes.

11. The method of claim 10, further comprising purifying nucleic acids from the lysed leukocytes.

12. The method of claim 10 or 11, further comprising analyzing nucleic acids from the lysed leukocytes.

13. The method of claim 12, comprising determining the total RNA from the lysed leukocytes.

14. The method of claim 12, comprising depleting mRNA from the lysed leukocytes.

15. The method of any one of claims 1-14, carried out using a multiwell device, the device having a plurality of wells, each well having a porous leukocyte depletion medium therein, wherein separate portions of biological fluid and wash fluid are passed through the leukocyte depletion media in separate wells.

16. The method of claim 8, further comprising administering the eluted leukocytes to a subject.
